# EUROPEAN PATENT SPECIFICATION

(11) **EP 1 854 404 B1**
(45) Date of publication and mention of the grant of the patent: **14.10.2009**
(21) Application number: 07107194.8
(22) Date of filing: 30.04.2007
(51) Int. Cl.: A61B 5/0476

(54) **Monitoring of the state of the central nervous system of a subject**
Überwachung des Zustands des zentralen Nervensystems einer Person
Surveillance de l'état du système nerveux central d'une personne

(30) Priority: 09.05.2006 US 431139
(43) Date of publication of application: 14.11.2007
(73) Proprietor: GE Healthcare Finland Oy, 00510 Helsinki (FI)
(72) Inventor: Sarkela, Mika, FIN-00400, Helsinki (FI)
(74) Representative: Bedford, Grant Richard

(56) References cited:
- EP-A- 1 468 646
- EP-A- 1 537 823
- WO-A-02/32305
- US-A1- 2006 079 801
- ANTON VAN BOXTEL ET AL: "Motor Unit Firing Rate During Static Contraction Indicated by the Surface EMG Power Spectrum" IEEE TRANSACTIONS ON BIOMEDICAL ENGINEERING, IEEE SERVICE CENTER, PISCATAWAY, NJ, US, vol. BME-19, no. 9, September 1983 (1983-09), pages 601-609, XP011173526 ISSN: 0018-9294

## Description

The present invention relates generally to the monitoring of the state of the central nervous system of a subject. The invention finds typical applications in processes monitoring a patient in connection with ICU (Intensive Care Unit) care or anesthesia.

Neuromonitoring is a subfield of clinical patient monitoring focused on measuring various aspects of brain function and on changes therein caused by drugs commonly used to induce and maintain anesthesia in an operation room or sedation in patients under critical or intensive care.

Electroencephalography (EEG) is a well-established method for assessing brain activity. When measurement electrodes are attached on the skin of the skull surface, the weak biopotential signals generated in the pyramid cells of the cortex may be recorded and analyzed. The EEG has been in wide use for decades in basic research of the neural systems of the brain as well as in the clinical diagnosis of various central nervous system diseases and disorders.

Electromyography (EMG) is a method for recording electrical biopotentials of muscles. In an EMG measurement, the electrodes are attached onto the surface of the skin overlying a muscle. When a biopotential signal is recorded from the forehead of a subject, the recorded signal indicates both the activity of the frontal muscles (fEMG) and the brain (EEG).

One of the special applications of the EEG, which has received attention recently, is the use of a processed EEG signal for objective quantification of the amount and type of brain activity for the purpose of determining the level of consciousness of a patient. In its simplest form, the utilization of an EEG signal allows automatic detection of the alertness of an individual, i.e. if he or she is awake or asleep. This has become an issue of increased interest, both scientifically and commercially, in the context of measuring the depth of unconsciousness induced by anesthesia during surgery.

Another important component of balanced anesthesia is analgesia which means prevention of pain reactions of a patient by administration of pain medication. Adequate analgesia reduces surgical stress and there is firm evidence that it decreases postoperative morbidity. Awareness during surgery with insufficient analgesia may lead to a post-traumatic stress disorder. Low quality pre- and intra-operative analgesia makes it difficult to select the optimal pain management strategy later on. More specifically, it may cause exposure to unwanted side effects during the recovery from a surgery. If the anesthesia is too light and involves insufficient hypnosis, it may cause traumatic experiences both for the patient and for the anesthesia personnel. From an economical point of view, if the anesthesia is too deep, it may cause increased perioperative costs through extra use of drugs and time, and extend the time required for post-operative care.

Virtually every patient being cared for in an ICU, for example, receives some form of sedation. However, the control of the depth of the sedation administered to a patient is still problematic, and therefore oversedation and undersedation are both common occurrences in intensive care units. At present, monitoring the level of sedation is mainly handled by using subjective observations from the patient. Various sedation assessment scales have been developed for subjectively assessing the level of sedation, the Ramsay Score being one of the most widely used tools for this purpose. Inappropriate sedation can lead to an adverse clinical outcome and reduce treatment efficacy in critical care settings. Oversedation may cause various complications, such as cardiovascular instability, and it may also increase the length of stay in the hospital and prolong the usage time of expensive facilities, such as the intensive care unit. Undersedation, in turn, may result in patient anxiety and agitation, which can further interfere with care, especially with that of neurological patients, and result in harm to the patient and the nursing staff.

In addition to the EEG signal data, EMG signal data obtained from facial muscles (fEMG) of the forehead is used for monitoring purposes during anesthesia and intensive care. Recovering frontal muscle activity is often the first indicator of the patient approaching consciousness. When this muscle activity is sensed by electrodes placed appropriately, it provides an early indication that the patient is emerging from anesthesia. Similarly, these electrodes can sense responses to nociceptive stimuli when the anesthesia is not adequate due to inadequate analgesia. So, the EMG signals give an early warning of arousal and may also be indicative of inadequate analgesia.

An objective tool for assessing the level of anesthesia or sedation is disclosed in U.S. Patent 6,801,803, which depicts a method and device for ascertaining the cerebral state of a patient. In this disclosure, a measure derived from EMG signal data enhances and confirms the determination of the hypnotic state made using EEG signal data. As the EMG data may be computed more frequently than the EEG data, it follows the changes in the hypnotic state of the patient more rapidly. The combined indicator provided by the EEG signal data and EMG signal data may also be used for assessing the adequacy of anesthesia or the level of sedation.

At present, the processes utilizing raw EEG signal data for monitoring a patient under sedation or anesthesia utilize the fact that the frequencies of the EMG spectrum are above the frequencies of brain activities, whereby the EMG components can be separated by methods of signal processing or spectral analysis from the EEG signal components contained in the signal data. Frontal muscle activity is then determined based on the signal power on a predefined frequency range corresponding to the EMG signal frequency band. However, current monitoring methods render acquisition of accurate information about frontal muscle activity cumbersome. This is mainly due to the fact that absolute EMG power varies significantly between different patients. Therefore, reference information about normal (typical) EMG power level of a particular patient is needed to obtain quantitative information about the current level of EMG activity.

One proposed solution to overcome this shortcoming of interpatient variability is to use the difference of so-called Response and State Entropies (RE and SE, respectively). Response and State Entropies are typically the spectral entropies derived from the frequency ranges of 0.8 to 47 Hz and 0.8 to 32 Hz, respectively.

Generally, the frequency range of SE covers the EEG component of the spectrum, whereas the frequency range RE also covers part of the EMG spectrum. The difference RE-SE is, therefore, indicative of the (f)EMG power, i.e. of frontal muscle activity. This generalization works well in a majority of the cases in clinical anesthesia routine. However, EMG may be in a dominant role already from the frequencies above 20 Hz, whereas EEG components may extend as high as up to about 80Hz. Therefore, the difference does not necessarily provide accurate information about frontal muscle activity for a larger group of subjects.

Preferred aspects of the present invention seek to alleviate or eliminate the above-mentioned drawback and to bring about a method by means of which the performance of EMG measurement may be improved in connection with monitoring of the state of the central nervous system of a subject.

Preferred aspects of the present invention seek to improve the performance of the EMG measurement in connection with monitoring of the state of the central nervous system of a subject. Preferred aspects of the invention further seek to provide a mechanism, which enables accurate and quantitative information to be obtained about the activity of facial muscles, especially of the activity of one or more of the frontal muscles, in connection with patient monitoring methods in which EMG activity is utilized to derive an indication of the state of the patient. A facial muscle here refers to a muscle in a facial area, i.e. in the non-hairy area of the head from the chin to the top of the forehead, whereas a frontal muscle refers to one of the muscles in a group including the frontalis, procerus, corrugator supercilii, and orbicularis muscles (i.e. the muscles on the forehead and around the eyes).

In Preferred aspects of the present invention, the EMG power measurement of the conventional monitoring methods is replaced by a measurement of the firing rate of at least one of the facial muscles. A two-dimensional electrode unit constructed to record motor unit action potentials from an underlying muscle is positioned on top of a facial muscle, especially a frontal muscle. Two-dimensionality here refers to the configuration of individual electrodes in the electrode unit: the electrode unit includes electrodes in two mutually orthogonal directions. Furthermore, the dimensions of the electrodes may vary in the third dimension. As discussed below, the electrode unit may be a distinct electrode unit or part of an electrode configuration comprising a plurality of electrode units. The firing rate of the at least one facial muscle is determined based on the recorded motor unit action potentials and the firing rate is employed to provide an indicator signal indicative of the state of the central nervous system of the subject. Depending on the application, the firing rate may be used as such as the indicator signal, or a combined indicator signal may be produced based on the firing rate and the EEG signal data acquired through other electrode units of the electrode configuration attached to the facial area of the subject. Generally speaking, the firing rate of a facial muscle is indicative of the state of the central nervous system. However, as is discussed below, in some cases the said indication may reflect the activity of the motor system only. This is the case, for example, when one or more other indicators measured simultaneously indicate that the cortical activity of the subject is low. The motor system here is a functional concept that refers to the physiological system responsible for the bodily functions acting through efferent nerves.

Thus one aspect of the invention is providing a method for monitoring the state of the central nervous system of a subject. The method includes the steps of positioning an electrode configuration onto a facial area of a subject, wherein the electrode configuration includes a multi-dimensional electrode unit constructed to record motor unit action potentials from an underlying facial muscle of the subject and acquiring biosignal data through the electrode configuration, wherein the biosignal data includes the motor unit action potentials acquired through the multi-dimensional electrode unit. The method further includes the steps of determining, based on the motor unit action potentials, a firing rate of the facial muscle of the subject and utilizing the firing rate as an indication of the state of the central nervous system of the subject.

Preferred aspects of the present invention enable accurate information to be acquired about the state of the central nervous system, especially of the state of the motor system of a subject, since the measured EMG activity indicates the said state without prior information about the normal EMG activity level of the subject. This is beneficial especially in ICU care where a newly arrived patient, about whom no prior measurement information is available, may be evaluated promptly and accurately in this respect.

Another aspect of the invention is that of providing an apparatus for monitoring the state of the central nervous system a subject. The apparatus includes an electrode configuration constructed to be positioned onto a facial area of a subject, the electrode configuration comprising a multi-dimensional electrode unit constructed to record motor unit action potentials from a facial muscle of a subject, first calculation means for determining, based on the motor unit action potentials, a firing rate of the facial muscle, and indicator means for providing an indicator signal indicative of the state of the central nervous system of the subject, wherein the indicator signal is dependent on the firing rate.

A further preferred aspect of the invention is that of providing a computer program product for monitoring the state of the central nervous system of a subject. The computer program product includes a first program code portion configured to receive a plurality of EMG signals, each EMG signal including a sequence of motor action potentials recorded from a facial muscle of a subject and a second program code portion configured to determine, based on the plurality of EMG signals, a firing rate of the facial muscle.

Other features and advantages of the invention will become apparent by reference to the following detailed description and accompanying drawings, in which:
FIG. 1 illustrates one embodiment of an electrode configuration for measuring facial EMG activity;
FIG. 2 is a flow diagram illustrating one embodiment of a method for monitoring a subject by the electrode configuration of FIG. 1;
FIG. 3 illustrates another embodiment of an electrode configuration for monitoring the state of the central nervous system of a subject;
FIG. 4 is a flow diagram illustrating one embodiment of a method for monitoring a subject by the electrode configuration of FIG. 3; and
FIG. 5 illustrates one embodiment of the apparatus or system of the invention.

In the human body, the muscular system includes three types of muscle tissues: smooth, striated (skeletal), and cardiac. Generally, striated muscles are voluntary, whereas smooth muscles and cardiac muscles are involuntary. The frontalis muscle, for example, is a striated muscle. Three main factors contribute to the amount of force of a striated muscle: the length of the muscle fibers, the thickness of the muscle, and the relative amount of red and white muscle fibers in the muscle cell. A motor unit is a functional unit that consists of a motoneuron and innervated muscle fibers. The combination of the muscle fiber action potentials from all the muscle fibers of a single motor unit is the motor unit action potential (MUAP). An individual MUAP train (MUAPT) propagating along a motoneuron can be modeled as a series of impulses. The frequency of the impulses in the MUAP train is called the firing rate. Marked differences in firing rate modulation exist among various striated muscles. Limb muscles regulate their force of contraction primarily by recruitment of active motor units, while facial muscles depend heavily on changes in the firing rate. It has been shown that the contraction strength is a linear function of the firing rate, cf. van Boxtel and Schomaker: Motor Unit Firing Rate During Static Contraction Indicated by the Surface EMG Power Spectrum, IEEE Transactions on Biomedical Engineering, vol. BME-30, No. 9, September 1983.

In preferred aspects of the present invention, the performance of the patient monitoring systems that rest on the measurement of EMG activity from the facial area is improved by determining the EMG activity through the firing rate of one or more of the facial muscles, especially one or more of the above-mentioned frontal muscles.

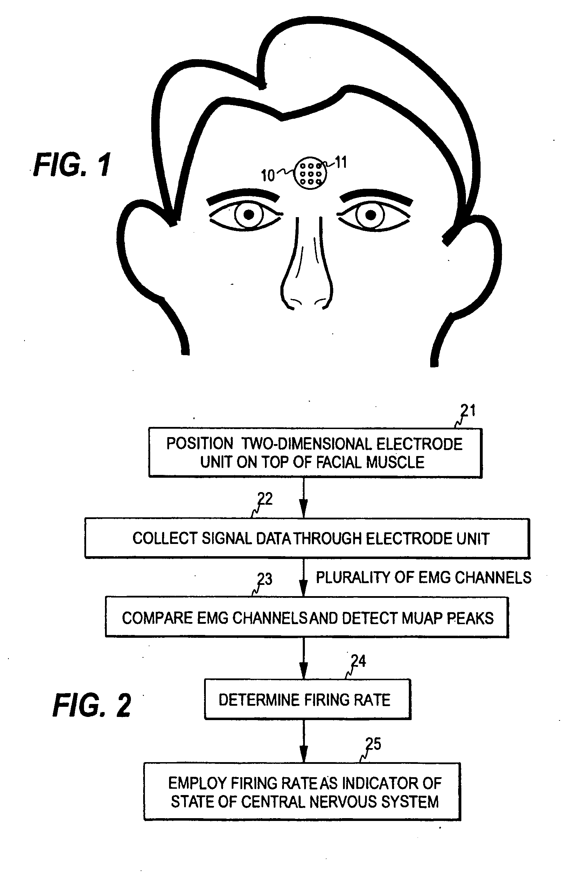

FIG. 1 illustrates one embodiment of the invention, which is useful in ICU care, for example. Preferred aspects of the present invention utilize a two-dimensional surface electrode unit 10, which is positioned on top of a facial muscle, especially on top of the frontalis or procerus muscle, as is shown in FIG. 1, to record the firing rate of the said muscle. The electrode unit includes a matrix ofNxN pin electrodes 11 for measuring the motor unit action potentials from an underlying muscle. The associated lead wires are not shown in FIG. 1.

A two-dimensional electrode array enables a user-friendly measurement, since the user does not have to place the electrodes in parallel with the muscle fibers, as would be the case if the motor unit action potentials were recorded through conventional surface or needle electrodes. The electrode arrangement of FIG. 1 may further include a ground electrode. One of the pin electrodes 11 may serve as the ground electrode, or a distinct ground electrode may be attached to the skin of the patient. Through the electrode unit, N channels may also be measured differentially. Furthermore, the lengths of the pin electrodes may vary, i.e. the pin electrodes may be at different depths in the tissue. Due to this, the electrode unit is also referred to as a multi-dimensional electrode unit below.

With reference to FIG. 2, a plurality of EMG signals is obtained from the patient (step 22) through the electrode unit 10 of FIG. 1. These signals are compared with each other to detect the motor unit action potential (MUAP) peaks (step 23). Based on the sequence of the detected peaks, the firing rate of the facial muscle may be determined (step 24). The firing rate may then be employed as an indicator indicative of the state of the central nervous system of the subject. The value of the firing rate is typically between 20 and 40 Hz, and the measured value may be used directly as the indicator. However, it is also possible to scale the measured firing rate to a desired output range. If a nonlinear scaling function is used to convert the measured firing rate values to the output values, the resolution of the measurement may be enhanced in the interesting region of the original firing rate scale. As discussed below, the indicator may be employed to reflect various aspects of the central nervous system, depending on whether other indicators of the state of the central nervous system are employed simultaneously.

Multi-channel EMG data measured through a multi-dimensional electrode array includes MUAP signals from several adjacent motor units. The firing rate of one motor unit is determined by comparing EMG data from the adjacent pin electrodes of the multi-dimensional electrode array. For this purpose, methods commonly used in image processing, for example spatial filtering, may be applied. Spatial filtering is typically carried out by convoluting the original two-dimensional signal with a two-dimensional filtering mask, which simultaneously enhances and attenuates certain parts of the signal. The two-dimensional mask may be based on a second-order derivative, in which case it is commonly called a Laplace mask.

Methods commonly used in time-domain signal analysis may also be used for determining the firing rate. For example, the independent component analysis or the principal components analysis may be used, or any other method which typically finds its application in blind signal separation. Furthermore, methods based on adaptive filtering may be applied. These methods combine spatial domain and time domain information. Before applying these methods, prefiltering may be carried out to eliminate possible artifacts and to decrease the amount of redundant information in the original signal data.

Algorithms combining different kind of signal processing methods may also be used. These algorithms may utilize information from conventional signal peak detectors and from other signal processing methods, such as some of the above-mentioned methods. The said algorithms may include decision tree structures and a priori set or adaptive threshold values.

An example of the determination of the firing rate is disclosed in Zhou P, Erim Z, Rymer WZ: Motor Unit Action Potential Counts in Surface Electrode Array EMG, Proceedings of the IEEE Engineering in Medicine and Biology Society, 25th Annual International Conference, Cancun, Mexico, September 17-23, 2003. pp 2067-2070. The article discloses a mechanism for measuring the firing rate of a hand muscle, and a similar mechanism may be utilized in connection with the monitoring method of certain embodiments of the present invention.

EMG activity is a sensitive indicator of the state of the central nervous system. However, as discussed above, EMG activity is not a specific indicator, but may be indicative of the cortical (i.e. hypnotic) state or of nociceptive responses, for example. It is further to be noted that the measured EMG activity also depends also on the condition of the peripheral nerves innervating the facial muscles. However, dysfunction of these nerves, which are cranial nerves emerging from the brainstem, is unusual. Furthermore, in anesthesia and in ICU care a clinician is primarily interested in monitoring the state of the central nervous system for which various preferred aspects of the present invention provide a non-invasive method. Therefore, if the rare occasion of the dysfunction of the cranial nerves concerned may be ruled out, the EMG activity provides a sensitive, but non-specific measure of the state of the central nervous system.

Due to the non-specificity, one or more further indicators may be utilized, which serve to improve the specificity of the EMG activity indicator. For example, if a further measure indicative of the hypnotic state is produced, the nursing staff may contemplate that whenever the measure of hypnotic state is low, increased EMG activity is indicative of activity in the motor system, acting through the neuronal pathways between the brainstem and the respective muscle from which the EMG activity is measured. During anesthesia, for example, this activity is typically induced by a nociceptive stimulus caused by a surgical procedure, for example.

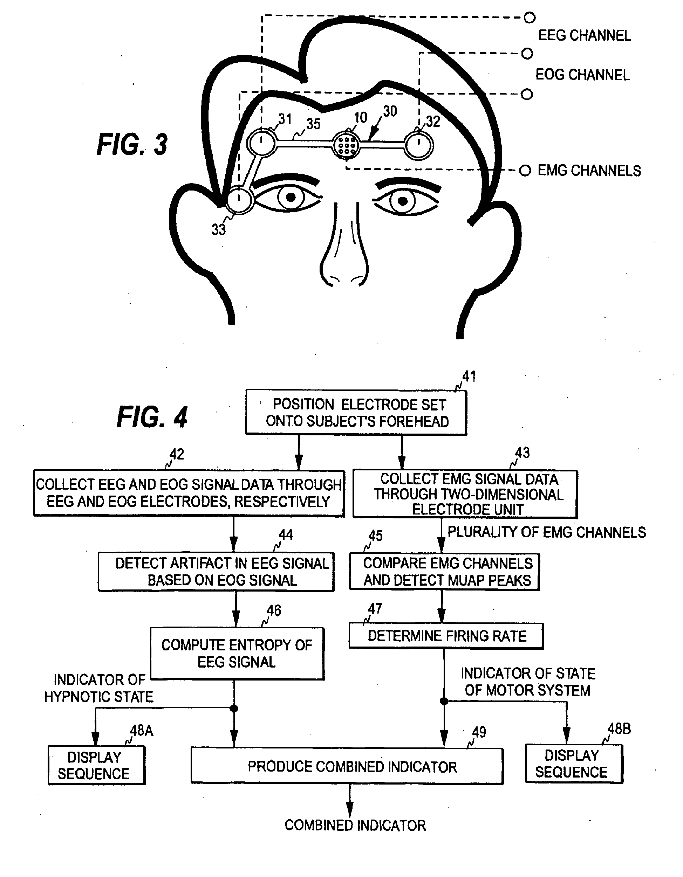

FIG. 3 and 4 illustrate embodiments in which a further indicator is produced to improve the specificity of the measured firing rate, and which may be employed for monitoring a patient during anesthesia, for example. In these embodiments, an electrode set comprising at least three electrodes is attached to the forehead of the patient, since at least two further electrodes are needed for recording EEG signal data from the subject.

FIG. 3 illustrates an electrode configuration 30 with four electrodes mounted on a thin and flexible substrate 35 made of plastic material, for example. As discussed below, the four-electrode configuration of FIG. 3 enables efficient detection of eye movement artefact in the EEG signal data. The electrode configuration comprises three conventional measurement electrodes 31 to 33 of which the first and second electrodes 31, 32 are for measuring the EEG signal and the third and second electrodes 33, 32 for measuring an electro-oculographic (EOG) signal. The four electrodes are integrated onto the surface of a strip-like substrate 35 comprising two portions; a first portion between electrodes 33 and 31 and a second portion between electrodes 31 and 32, the first portion being set to an angle relative to the second portion. A two-dimensional EMG electrode 10 according to FIG. 1 lies substantially at the center line between the first and second electrodes.

The nursing staff may first attach the EMG electrode on top of the frontalis or procerus muscle by aligning the said electrode with the vertical center line of the face, for example. The first and second electrodes 31 and 32 may then be positioned onto the hairless fronto-lateral areas of the frontal lobe of the patient, preferably as far as possible from the eye. The electrodes are positioned similarly, but on the opposite cortical hemispheres of the patient. Furthermore, the first and second electrodes are positioned substantially horizontally (i.e. in the same horizontal line) and at substantially equal distances from the vertical center axis of the face, i.e. the second measurement electrode is positioned onto the spot which is the mirror image of the spot of the first measurement electrode, and vice versa, the vertical center axis being the mirror axis.

The EEG signal is measured from the first and second measurement electrodes 31 and 32. Due to the symmetrical positions of the electrodes, the potential changes caused by vertical eye movements are substantially the same at both electrodes (assuming that the eyes move similarly to each other, as is the case normally). In other words, the potential changes caused by vertical eye movements tend to cancel in the EEG signal, which is represented by the voltage difference of the electrodes. However, the EEG channel remains sensitive to horizontal eye movements. In the embodiment of FIG. 3, a third measurement electrode 33 and one of the first and second measurement electrodes are employed to measure the EOG signal. For this purpose, the third measurement electrode is positioned so that the EOG voltage measured between this electrode and one of the first and second electrodes is as high as possible in case of horizontal movement of the eyes. In the example of FIG. 3, the said one electrode is the second measurement electrode 32, i.e. the EOG voltage is measured between electrodes 33 and 32. Therefore, the third measurement electrode is positioned on the temple of the patient, on the hemisphere opposite to that of measurement electrode 32. The temple here refers to the area between an eye and the ear on the same hemisphere as the eye. Various other electrode configurations that enable the detection of eye movements with a minimum number of electrodes are disclosed in Applicant's U.S. Patent Application No. 11/316,374. However, it is to be noted that the EOG channel is not necessary for producing the indicator signals indicative of the state of the central nervous system of a subject, but a single EEG channel is enough for this purpose, together with the plurality of EMG channels provided by the two-dimensional EMG electrode.

FIG. 4 illustrates the measurement method in connection with the electrode arrangement of FIG. 3. The biosignals obtained from the electrodes (cf. steps 41 to 43) are first digitized and the digitized signals are divided into successive epochs. As is common in the art, the digitized signal samples are processed as sets of sequential signal samples representing finite time blocks or time windows, commonly termed "epochs".

As mentioned above, the EOG channel is in this embodiment utilized to detect eye movement artifact in the EEG signal data (step 44). This may be carried out, for example, by comparing, for each epoch, the EEG and EOG signals with each other. As is discussed in the above-mentioned U.S. Patent Application, the comparison may involve the determination of the difference of the absolute values of simultaneous signal values. If any of the differences within an epoch exceeds a predetermined threshold, the epoch is flagged to indicate that it is contaminated by eye movement artifact.

Based on the pre-processed EEG signal data obtained from step 44, the entropy values of the EEG signal data are calculated in successive time windows (epochs). In this example, entropy refers to spectral entropy, i.e. the pre-processed EEG signal data is subjected to a spectral decomposition, which may be carried out, for example, by a Fourier transform. However, several other types of entropies may also be utilized, such as Shannon entropy or approximate entropy. Furthermore, instead of different types of entropies step 46 may include the determination of a parameter related to the amount of irregularity in the EEG signal data. Other possible quantifications that may be used in this context include fractal spectrum analysis, Lempel-Ziv complexity, or spectral, bispectral, multispectral or stationarity analyses. Consequently, step 46 outputs a sequence of a parameter indicative of the irregularity of the EEG signal data and thus also of the hypnotic state of the subject.

The output of step 46 thus serves as an indicator of the hypnotic state of the patient, reflecting the activity of the cortical areas of the patient. The sequence of the indicator may be supplied to a display device and thus presented to the nursing staff (step 48A).

Simultaneously with the above steps, the firing rate is determined based on the EMG signal data obtained from the plurality of the pin electrodes, cf. steps 43, 45, and 47 which correspond, respectively, to steps 22, 23, and 24 of FIG. 1. As discussed above, in this application the firing rate serves mainly as an indicator of the state of the motor system, whereas the entropy-based indicator is indicative of cortical activity. The firing rate sequence may also be supplied to a display device and presented to the nursing staff (step 48B).

The indicator sequence obtained from step 46 and the firing rate sequence obtained from step 47 may further be employed to produce a composite indicator, which is indicative of the state of the central nervous system of the subject (step 49). The composite indicator may be calculated as a weighted sum of the entropy and firing rate values that relate temporally to each other, whereby a sequence of the composite indicator is obtained from step 49.

It is also possible that the firing rate values and/or the entropy values are scaled or normalized prior to the respective display steps 48A and 48B and/or prior to the production of the composite indication. Furthermore, the production of the composite indication may simply involve presenting the two simultaneous values to the user of the monitoring device so that the user may assess their relative magnitudes. For example, two columns may be displayed to the user, the height of the first column indicating the value of the EEG indicator (entropy) and the height of the second column indicating the value of the EMG activity indicator. The nursing staff may then evaluate the state of the patient based on the total and relative heights of the two columns. In this case, steps 48A and 48B are thus combined to form step 49.

In an ICU environment, the objective of the EEG measurement is typically to diagnose the patient, whereas in anesthesia the objective is typically to monitor the hypnotic state of the patient. Due to the difference, ICU care less frequently involves an EEG measurement from the frontal area of the patient, but in ICU care the EEG is more often measured for diagnostic purposes with a greater number of electrodes positioned onto various areas of the whole scalp. Therefore, the embodiments of FIG. 3 and 4 are not as useful in ICU care as in anesthesia. However, the embodiments of FIG. 1 and 2 are advantageous in ICU care, since the measured EMG activity provides quantitative information of the state of the central nervous system without prior reference information, which is required if the EMG activity is measured through EMG power.

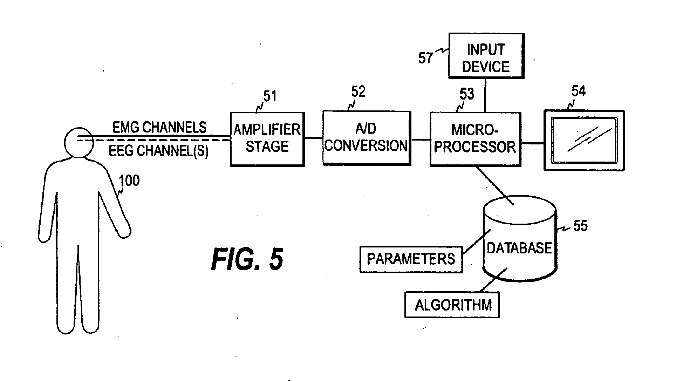

FIG. 5 illustrates one embodiment of the system or apparatus according to the invention. The physiological signals, i.e. the EMG signals and the optional biopotential signal(s), obtained through an electrode arrangement attached to the facial area of a subject 100 are supplied to an amplifier stage 51, which amplifies the signal(s) before they are sampled and converted into digitized format in an A/D converter 52. The digitized signals are supplied to a computer unit 53 which may comprise one or more processors. The signal path between the electrodes and the computer unit may also be provided with various pre-processing stages, such as filtering stages, which serve to remove non-idealities from the measured signals.

The computer unit is provided with a memory or database 55, which may hold the digitized signal data obtained from the electrodes and the algorithm for producing the indicator signal(s) of preferred aspects of the invention. Using the stored algorithm the computer unit executes the functions described above in order to obtain the indicator sequence(s). The term computer unit here refers to any system, processor, circuit, or computing entity which is capable of computing the indicator(s) based on the biosignal data obtained from a subject.

The user may supply information, such as the parameters required by the algorithm, through a user input device 57. The computer unit may display the results, i.e. the indicator signal(s), through at least one monitor 54 connected to the computer unit.

Although one computer unit or processor may perform the above steps, the processing of the data may also be distributed among different units/processors (servers) within a network, such as a hospital LAN (local area network). The apparatus of certain embodiments of the invention may thus also be implemented as a distributed system. However, the implementation of the apparatus as a compact monitoring unit, which may be movable with the patient, allows the monitoring of the patient to be continued in a post anesthetic care unit, for example, or in a non-hospital setting.

An existing patient monitor may also be upgraded to enable the monitor to compute the indicator signal(s) according to preferred aspects of the invention. Such an upgrade may be implemented by delivering to the patient monitor a software module that enables the monitor to define the firing rate of a facial muscle based on the EMG signals received from a two-dimensional EMG electrode. The software module may be delivered, for example, on a data carrier, such as a CD or a memory card, or through a telecommunications network. The software module may receive the biosignal data in real-time directly from the corresponding electrodes or from the memory of the patient monitor after the actual measurement of the data. In the latter case, the signals may already be temporally aligned by time stamps attached to the signal values. In this case the software module may also be in a device which may not be able to perform the actual measurement, but only the calculation of the results after the actual measurement is performed by another device.

The method of preferred aspects of the invention may also be used for monitoring subjects outside a hospital environment. For example, the level of alertness of people working in professions requiring sustained attention and/or concentration may be monitored to obtain an early warning of fatigue. Such systems may be used, for example, for preventing professional drivers from dozing off while driving. The method of the invention may also be used for monitoring the level of sedation of elderly people living in nursing homes, for example.

A further interesting field of application is the diagnosis and/or monitoring of migraine or headache patients. International headache classification recommends evaluation of EMG levels, especially quantitative EMG levels, in pericranial muscles of headache patients. As is discussed above, preferred aspects of the present invention are especially beneficial for the determination of quantitative EMG information from the said muscles. In monitoring a headache patient, an ambulatory monitoring device may be used, since headache patients tend to have increased EMG levels during both awake and sleep periods.

Although the invention was described above with reference to the examples shown in the appended drawings, it is clear that the invention is not limited to these, but may be modified by those skilled in the art without departing from the scope of the invention. For example, the EMG activity may be derived from a plurality of the frontal muscles with a corresponding plurality of two-dimensional electrode units.

## Claims

1. A method for monitoring the state of the central nervous system of a subject, the method comprising the steps of:
- positioning an electrode configuration (10; 30) onto a facial area of a subject (100), wherein the electrode configuration includes a multi-dimensional electrode unit (10) constructed to record motor unit action potentials from an underlying facial muscle of the subject;
- acquiring (22) biosignal data through the electrode configuration, wherein the biosignal data includes the motor unit action potentials acquired through the multi-dimensional electrode unit;
**characterised by**
- determining (24), based on the motor unit action potentials, a firing rate of the facial muscle of the subject; and
- utilizing (25) the firing rate as an indication of the state of the central nervous system of the subject.

2. A method according to claim 1, wherein the positioning step includes positioning the multi-dimensional electrode on top of a frontal muscle of the subject.

3. A method according to claim 1 or claim 2, wherein
- the positioning step includes positioning the electrode configuration onto the facial area, wherein the electrode configuration further includes EEG electrodes; and
- the acquiring step includes acquiring the biosignal data, in which the biosignal data further includes EEG signal data acquired through the EEG electrodes.

4. A method according to any preceding claim, wherein the utilizing step includes providing the firing rate as an indication of the state of the central nervous system of the subject.

5. A method according to claim 3 or claim 4 when dependent thereon, further comprising a step of deriving a measure of the irregularity of the EEG signal data.

6. A method according to claim 5, wherein the utilizing step includes producing (49) a composite indication from the firing rate and the measure of the irregularity, the composite indication being indicative of the state of the central nervous system of the subject.

7. A method according to claim 5 or claim 6, further comprising a step of displaying (48B, 48A) the firing rate and the measure of the irregularity.

8. A method according to any preceding claim, wherein at least the positioning, acquiring, and utilizing steps are performed in an Intensive Care Unit (ICU) or in an operating room.

9. A method according to any preceding claim, wherein the positioning, acquiring, determining, and utilizing steps are performed in a non-hospital setting.

10. A method according to any preceding claim, wherein the utilizing step includes utilizing the firing rate as the indication of the state of the central nervous system of the subject, in which the subject is a headache patient.

11. An apparatus for monitoring the state of the central nervous system of a subject, the apparatus comprising:
- an electrode configuration (10; 30) constructed to be positioned onto a facial area of a subject (100), the electrode configuration comprising a multi-dimensional electrode unit (10) constructed to record motor unit action potentials from a facial muscle of a subject;
**characterised by**
- first calculation means (53) for determining, based on the motor unit action potentials, a firing rate of the facial muscle; and
- indicator means (53, 54) for providing an indicator signal indicative of the state of the central nervous system of the subject, wherein the indicator signal is dependent on the firing rate.

12. An apparatus according to claim 11, wherein
- the electrode configuration further comprises EEG electrodes for acquiring EEG signal data; and
- the apparatus includes second calculation means (53) for deriving a measure of the irregularity of the EEG signal data.

13. An apparatus according to claim 11 or Claim 12, wherein the indicator means comprise a display unit (54) configured to display the firing rate as the indicator signal indicative of the state of the central nervous system of the subject.

14. An apparatus according to any one of claims I 1 to 13, wherein the indicator means comprise
- third calculation means (53) configured to calculate a composite indication from the firing rate and the measure; and
a display unit (54) configured to display the composite indication as the indicator signal indicative of the state of the central nervous system of the subject.

15. An apparatus according to any one of claims 11 to 14, wherein the multi-dimensional electrode unit (10; 30) comprises a plurality of pin electrodes (11) arranged in the form of a matrix.

## Patentansprüche

1. Verfahren zur Überwachung des Zustands des zentralen Nervensystems einer Person, umfassend die Schritte:
- Positionierung einer Elektrodenanordnung (10; 30) auf einem Gesichtbereich einer Person (100), wobei die Elektrodenanordnung eine multidimensionale Elektrodeneinheit (10) umfasst, welche zur Aufnahme von Aktionspotentialen motorischer Einheit von einem unterliegenden Gesichtsmuskel der Person ausgebildet ist;
- Aufnahme (22) von Biosignaldaten über die Elektrodenanordnung, wobei die Biosignaldaten die durch die multidimensionale Elektrodeneinheit erhaltenen Aktionspotentiale motorischer Einheit beinhalten;
**gekennzeichnet durch**
- das Bestimmen (24) einer Entladungsrate des Gesichtsmuskels der Person basierend auf den Aktionspotentialen motorischer Einheit; und
- die Verwendung (25) der Entladungsrate als ein Anzeichen für den Zustand des zentralen Nervensystems der Person.

2. Verfahren nach Anspruch 1, wobei der Positionierungsschritt die Positionierung der multidimensionalen Elektrode auf einem Stirnflächenmuskel der Person umfasst.

3. Verfahren nach Anspruch 1 oder 2, wobei
- der Positionierungsschritt die Positionierung der Elektrodenanordnung auf dem Gesichtsbereich umfasst, wobei die Elektrodenanordnung weiter EEG-Elektroden umfasst; und
- der Aufnahmeschritt die Aufnahme der Biosignaldaten umfasst, in welchen die Biosignaldaten weiter durch die EEG-Elektroden erhaltene EEG-Signaldaten umfassen.

4. Verfahren nach einem der vorhergehenden Ansprüche, wobei der Verwendungsschritt die Bereitstellung der Entladungsrate als ein Anzeichen des Zustands des zentralen Nervensystems der Person umfasst.

5. Verfahren nach Anspruch 3 oder 4, wenn davon abhängig, weiter umfassend einen Schritt der Ableitung eines Maßes der Abweichungen der EEG-Signaldaten.

6. Verfahren nach Anspruch 5, wobei der Verwendungsschritt die Erzeugung (49) eines aus der Entladungsrate und dem Maß der Abweichungen zusammengesetzten Anzeichen umfasst, wobei das zusammengesetzte Anzeichen für den Zustand des zentralen Nervensystems der Person bezeichnend ist.

7. Verfahren nach Anspruch 5 oder 6, weiter umfassend einen Schritt der Anzeige (48B, 48A) der Entladungsrate und des Maßes der Abweichungen.

8. Verfahren nach einem der vorhergehenden Ansprüche, wobei wenigstens der Positionierungs-, der Aufnahme-, der Bestimmungs- und der Verwendungsschritt in einer Intensivmedizinstation oder in einem Operationssaal durchgeführt werden.

9. Verfahren nach einem der vorhergehenden Ansprüche, wobei der Positionierungs-, der Aufnahme-, der Bestimmungs- und der Verwendungsschritt in einem nicht-klinischen Umfeld durchgeführt werden.

10. Verfahren nach einem der vorhergehenden Ansprüche, wobei der Verwendungsschritt die Verwendung der Entladungsrate als das Anzeichen für den Zustand des zentralen Nervensystems der Person umfasst, wobei die Person ein Kopfschmerzpatient ist.

11. Vorrichtung zur Überwachung des Zustands des zentralen Nervensystems einer Person, umfassend:
- eine zur Positionierung auf einem Gesichtbereich einer Person (100) ausgebildete Elektrodenanordnung (10; 30), wobei die Elektrodenanordnung eine multidimensionale Elektrodeneinheit (10) umfasst, welche zur Aufnahme von Aktionspotentialen motorischer Einheit von einem unterliegenden Gesichtsmuskel einer Person ausgebildet ist;
**gekennzeichnet durch**
- erste Berechnungsmittel (53) zur Bestimmung einer Entladungsrate des Gesichtsmuskels basierend auf den Aktionspotentialen motorischer Einheit; und
- Anzeichenmittel (53, 54) zur Bereitstellung eines für den Zustand des zentralen Nervensystems der Person bezeichnenden Anzeichensignals, wobei das Anzeichensignal von der Entladungsrate abhängig ist.

12. Vorrichtung nach Anspruch 12, wobei
- die Elektrodenanordnung weiter EEG-Elektroden zur Aufnahme von EEG-Signaldaten umfasst; und
- die Vorrichtung zweite Berechnungsmittel (53) zur Ableitung eines Maßes der Abweichungen der EEG-Signaldaten umfasst.

13. Vorrichtung nach Anspruch 11 oder 12, wobei die Anzeichenmittel eine zur Anzeige der Entladungsrate als ein Anzeichen des Zustands des zentralen Nervensystems der Person ausgebildete Anzeigeeinheit (54) umfasst.

14. Vorrichtung nach einem der Ansprüche 11 bis 13, wobei die Anzeichenmittel
- dritte Berechnungsmittel (53), welche zur Berechnung eines aus der Entladungsrate und dem Maß zusammengesetzten Anzeichens ausgebildet sind; und
- eine Anzeigeeinheit (54) aufweist, welche zur Anzeige des zusammengesetzten Anzeichens als dem für den Zustand des zentralen Nervensystems der Person bezeichnenden Anzeichensignal ausgebildet ist.

15. Vorrichtung nach einem der Ansprüche 11 bis 14, wobei die multidimensionale Elektrodeneinheit (10; 30) eine Anzahl in Form einer Matrix angeordnete Pinelektroden (11) umfasst.

## Revendications

1. Procédé de surveillance de l'état du système nerveux central d'un sujet, le procédé comprenant les étapes suivantes :
- positionnement d'une configuration d'électrodes (10 ; 30) sur une zone faciale d'un sujet (100), la configuration d'électrodes comprenant une unité d'électrode multidimensionnelle (10) construite pour enregistrer les potentiels d'action d'unité motrice d'un muscle facial sous-jacent du sujet ;
- acquisition (22) de données de signal biologique par l'intermédiaire de la configuration d'électrodes, les données de signal biologique incluant les potentiels d'action d'unité motrice acquis par l'intermédiaire de l'unité d'électrode multidimensionnelle ;
**caractérisé par** :
- la détermination (24), d'après les potentiels d'action d'unité motrice, d'une fréquence de décharge du muscle facial du sujet; et
- l'utilisation (25) de la fréquence de décharge comme indication de l'état du système nerveux central du sujet.

2. Procédé selon la revendication 1, dans lequel l'étape de positionnement comprend le positionnement de l'électrode multidimensionnelle au sommet d'un muscle frontal du sujet.

3. Procédé selon la revendication 1 ou 2, dans lequel :
- l'étape de positionnement comprend le positionnement de la configuration d'électrodes sur la zone faciale, la configuration d'électrodes comprenant en outre des électrodes d'E.E.G. ; et
- l'étape d'acquisition comprend l'acquisition des données de signal biologique, les données de signal biologique comprenant en outre des données de signal d'E.E.G. acquises au moyen des électrodes d'E.E.G.

4. Procédé selon l'une quelconque des revendications précédentes, dans lequel l'étape d'utilisation comprend le fait de fournir la fréquence de décharge comme indication de l'état du système nerveux central du sujet.

5. Procédé selon la revendication 3 ou 4 lorsqu'elle dépend de celle-ci, comprenant en outre une étape consistant à dériver une mesure de l'irrégularité des données de signal d'E.E.G.

6. Procédé selon la revendication 5, dans lequel l'étape d'utilisation comprend la production (49) d'une indication composée à partir de la fréquence de décharge et de la mesure de l'irrégularité, l'indication composée étant représentative de l'état du système nerveux central du sujet.

7. Procédé selon la revendication 5 ou 6, comprenant en outre une étape d'affichage (48B, 48A) de la fréquence de décharge et de la mesure de l'irrégularité.

8. Procédé selon l'une quelconque des revendications précédentes, dans lequel au moins les étapes de positionnement, d'acquisition et d'utilisation sont exécutées dans une Unité de Soins Intensifs (USI) ou dans une salle d'opération.

9. Procédé selon l'une quelconque des revendications précédentes, dans lequel les étapes de positionnement, d'acquisition, de détermination et d'utilisation sont exécutées dans un environnement non hospitalier.

10. Procédé selon l'une quelconque des revendications précédentes, dans lequel l'étape d'utilisation comprend le fait d'utiliser la fréquence de décharge comme indication de l'état du système nerveux central du sujet, le sujet étant un patient souffrant de céphalée.

11. Appareil permettant de surveiller l'état du système nerveux central d'un sujet, l'appareil comprenant :
- une configuration d'électrodes (10 ; 30) destinée à être positionnée sur une zone faciale d'un sujet (100), la configuration d'électrodes comprenant une unité d'électrode multidimensionnelle (10) construite pour enregistrer les potentiels d'action d'unité motrice d'un muscle facial d'un sujet ;
**caractérisé par** :
- un premier moyen de calcul (53) pour déterminer, d'après les potentiels d'action d'unité motrice, une fréquence de décharge du muscle facial ; et
- un moyen d'indication (53, 54) pour fournir un signal indicateur représentant l'état du système nerveux central du sujet, le signal indicateur dépendant de la fréquence de décharge.

12. Appareil selon la revendication 11, dans lequel :
- la configuration d'électrodes comprend en outre des électrodes d'E.E.G. pour acquérir des données de signal d'E.E.G. ; et
- l'appareil comprend un deuxième moyen de calcul (53) pour dériver une mesure de l'irrégularité des données de signal d'E. E. G.

13. Appareil selon la revendication 11 ou 12, dans lequel le moyen d'indication comprend une unité d'affichage (54) configurée pour afficher la fréquence de décharge comme signal indicateur représentant l'état du système nerveux central du sujet.

14. Appareil selon l'une quelconque des revendications 11 à 13, dans lequel le moyen d'indication comprend :
- un troisième moyen de calcul (53) configuré pour calculer une indication composée à partir de la fréquence de décharge et de la mesure ; et
- une unité d'affichage (54) configurée pour afficher l'indication composée comme signal indicateur représentant l'état du système nerveux central du sujet.

15. Appareil selon l'une quelconque des revendications 11 à 14, dans lequel l'unité d'électrode multidimensionnelle (10 ; 30) comprend une pluralité d'électrodes du type broche (11) disposées de manière à former une matrice.
